(19) Europäisches Patentamt
European Patent Office
Office européen des brevets

(11) Numéro de publication : **0 195 730**
**B1**

(12) # FASCICULE DE BREVET EUROPÉEN

(45) Date de publication du fascicule du brevet : **15.03.89**

(51) Int. Cl.[4] : **A 23 L 1/237**

(21) Numéro de dépôt : **86420071.2**

(22) Date de dépôt : **13.03.86**

(54) **Nouveaux agents édulcorants dérivant de la glycine et de la bêta-alanine, procédé pour édulcorer des produits divers et compositions contenant de tels agents édulcorants.**

(30) Priorité : **19.03.85 FR 8504242**

(43) Date de publication de la demande : **24.09.86 Bulletin 86/39**

(45) Mention de la délivrance du brevet : **15.03.89 Bulletin 89/11**

(84) Etats contractants désignés : **AT BE CH DE FR GB IT LI LU NL SE**

(56) Documents cités :
**FR–A– 2 182 921**
**CHEMICAL ABSTRACTS, vol. 82, no. 21, 26 mai 1975, page 623, résumé no. 140061p, Columbus, Ohio, US; Y. YUKI et al.: "Preparations and reactions of dihydropyrimidines. I. Reactions of urea, thiourea, and S-ethylisothiourea with methyl acrylate"**

(73) Titulaire : **UNIVERSITE CLAUDE BERNARD - LYON 1**
**86 rue Pasteur**
**F-69007 Lyon (FR)**

(72) Inventeur : **Nofre, Claude**
**119 cours Albert Thomas**
**F-69003 Lyon (FR)**
Inventeur : **Tinti, Jean Marie**
**5 avenue de Grenoble**
**F-69330 Meyzieu (FR)**
Inventeur : **Ouar, Farroudja**
**épouse Chatzopoulos 14, rue Raoul Follereau**
**F-42100 Saint-Etienne (FR)**

(74) Mandataire : **Laurent, Michel et al**
**Cabinet LAURENT et GUERRE B.P. 32**
**F-69131 Ecully Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 195 730 B1

**Description**

L'invention concerne de nouveaux agents édulcorants. Elle vise l'utilisation de ces composés pour édulcorer les aliments, les boissons, les confiseries, les chewing gums, les produits d'hygiène, les cosmétiques, les produits pharmaceutiques et vétérinaires, etc. Enfin, elle couvre les produits et les compositions contenant de tels agents édulcorants.

Parmi les composés chimiques qui présentent des propriétés édulcorantes, le « suosan » et ses dérivés constituent une série chimique qui a été largement étudiée depuis leur découverte en 1948 par Petersen et Müller (voir par exemple Beets, Structure-Activity Relationships in Human Chemoreception, Applied Science Publ., London, 1978, pp. 336-337 ; Crosby et Wingard, in Developments in Sweeteners, Applied Science Publ., London 1979, p. 160 ; Tinti, Nofre et Peytavi, Z. Lebensm. Unters. Forsch., 1982, 175, 266-268). Mais ces composés n'ont jamais été utilisés en pratique car certains d'entre eux libèrent des molécules potentiellement toxiques ; c'est le cas du « suosan » qui conduit à de la p-nitroaniline. Enfin, certains de ces composés présentent, à côté de leur goût sucré, un arrière-goût réglissé ou amer.

L'invention pallie ces inconvénients.

Elle concerne des agents édulcorants qui se caractérisent en ce qu'ils répondent à la formule générale :

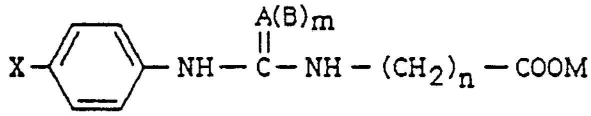

dans laquelle :

— A est un groupe imino (= N—), iminium ($=\overset{+}{N}<$ ), méthylène (=C < ), le groupe iminium pouvant être salifié par un anion organique ou inorganique physiologiquement acceptable ;

— m et n sont des nombres égaux à 1 ou 2 ;

— B :

quand n = 1, représente H, CN, $OCH_3$, $NO_2$, $SO_2R$, SOR, $SO_2NHR$, $SO_2NR_2$, où R est un groupe alkyle, cycloalkyle ou aryle ayant jusqu'à 10 atomes de carbone, 1 ou 2 atomes de carbone pouvant être substitués par 1 ou 2 atomes de soufre ou d'oxygène ;

quand n = 2, représente H, CN, $OCH_3$ ;

— X :

représente CN, $NO_2$, quand B est H, CN, $OCH_3$ ;

représente CN, $NO_2$, $COCH_3$, CHO, Cl, $CF_3$, F, H, quand B est $NO_2$, $SO_2R$, SOR, $SO_2NHR$, $SO_2NR_2$ ;

— M est un atome d'hydrogène ou un cation organique ou inorganique physiologiquement acceptable.

Par anion ou cation « physiologiquement acceptable », on entend tout anion ou cation qui ne présente pas de toxicité caractérisée pour l'organisme.

Dans une forme de réalisation préférée :

— A est un groupe imino (= N—), iminium ($=\overset{+}{N}<$ ), méthylène (= C < ), le groupe iminium pouvant être salifié par un ion chlorure ;

— B :

quand n = 1, représente H, CN, $OCH_3$, $NO_2$, $SO_2R$, où R est un groupe alkyle normal ou ramifié jusqu'à 5 atomes de carbone, isohexyle, phényle ($C_6H_5$), cyclohexyle ($C_6H_{11}$), benzyle ($C_6H_5CH_2$), tolyle ($CH_3C_6H_4$), cyclohexylméthyle ($C_6H_{11}CH_2$) ;

quand n = 2, représente H, CN, $OCH_3$ ;

— X est CN ;

— M est un atome d'hydrogène ou un cation pris dans le groupe $Na^+$, $K^+$, $NH_4^+$, 1/2 $Ca^{2+}$, 1/2 $Mg^{2+}$.

Avantageusement, en pratique :

— l'agent édulcorant est caractérisé en ce qu'il consiste en un composé de formule :

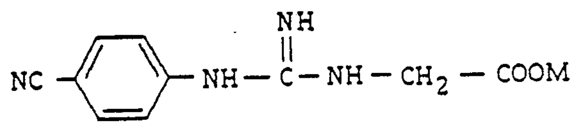

— l'agent édulcorant est caractérisé en ce qu'il consiste en un composé de formule :

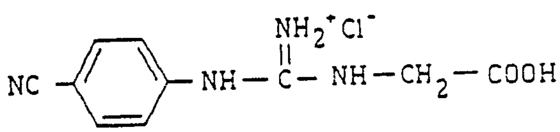

— l'agent édulcorant est caractérisé en ce qu'il consiste en un composé de formule :

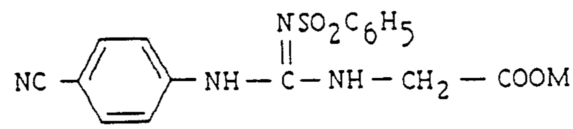

— l'agent édulcorant est caractérisé en ce qu'il consiste en un composé de formule :

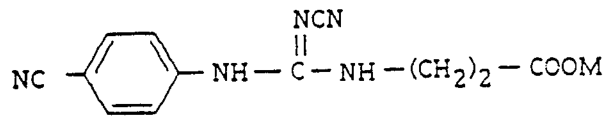

En d'autres termes, l'invention concerne des dérivés perfectionnés du « suosan » qui se caractérisent notamment par le remplacement des groupes uréido ou thiouréido par des groupes guanidino, guanidinium ou 1,1-diaminoéthényle, ces groupes étant éventuellement substitués par les groupes CN, $OCH_3$, $NO_2$, $SO_2R$, SOR, $SO_2NHR$, $SO_2NR_2$.

Ce perfectionnement présente, de manière inattendue, l'avantage non seulement de conserver une saveur sucrée à ces nouveaux composés, mais aussi d'exalter très souvent le pouvoir édulcorant par rapport à celui des dérivés uréido ou thiouréido, puisqu'on obtient, par ce perfectionnement, des composés qui sont jusqu'à 45 000 (quarante cinq mille) fois plus sucrés que le saccharose (sucrose).

Dans les Chemical Abstracts (vol. 82, 1975, n° 140061p), il est cité un article de Y. Yuki et K. Inoue (Nippon Kagaku Kaishi, 1974, n° 11, 2140-3) où on trouve décrit la N-[(4-chlorophénylamino)iminométhyl]-β-alanine (Chemical Substance Index, vol. 76-85, 1972-1976, p. 1067cs) ; ce dérivé chloré, contrairement aux composés de l'invention, ne possède aucune propriété édulcorante, ce qui est la preuve du caractère imprévisible de l'invention.

Le perfectionnement de la présente invention permet aussi de supprimer les arrière-goûts amers ou réglissés que présentent souvent les dérivés uréido ou thiouréido correspondants. Il n'est pas en effet possible de différencier du saccharose, par les seuls caractères organoleptiques, les nouveaux composés ainsi obtenus.

L'invention concerne également le procédé qui consiste à édulcorer un produit en lui ajoutant un tel agent édulcorant en quantité efficace ; elle concerne aussi le produit ainsi édulcoré. Par « quantité efficace », on désigne une quantité qui soit détectable par les sensations physiologiques d'un être humain. Elle concerne enfin toute composition comprenant un tel agent édulcorant associé à un support compatible ou à un autre agent édulcorant.

Les composés selon l'invention peuvent être préparés par condensation entre des composés de formules générales suivantes :

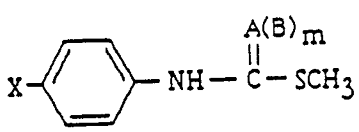
$H_2N-(CH_2)_n-COOM$

La réaction entre les deux composés peut être effectuée en présence d'une base, la base étant choisie dans le groupe comprenant l'hydroxyde de sodium, l'hydroxyde de potassium, ou une amine tertiaire comme, par exemple, la triéthylamine. La condensation est effectuée à ébullition dans un mélange éthanol-eau.

Le pouvoir sucrant des composés a été évalué par un groupe de six goûteurs expérimentés. Pour cela, les composés, en solution aqueuse à des concentrations variables, sont comparés, sur le plan gustatif, à des solutions témoins de saccharose à des concentrations, respectivement, de 2 %, 5 % et 10 %, c'est-à-dire à des concentrations correspondant à celles utilisées lors d'un usage courant ; le pouvoir sucrant des édulcorants de synthèse varie en effet suivant la concentration de la solution de saccharose utilisée comme référence. Le pouvoir sucrant du composé testé par rapport au saccharose correspond alors au rapport pondéral qui existe entre le composé et le saccharose à l'isosucrosité, c'est-à-dire quand les saveurs sucrées entre la solution du composé testé et la solution témoin de saccharose sont considérées, par une majorité de goûteurs, avoir le même pouvoir sucrant.

La manière dont l'invention peut être réalisée et les avantages qui en découlent ressortiront mieux des exemples de réalisation qui suivent.

## Exemple 1

Synthèse de l'acide N-[cyanoimino(4-cyanophényl-amino)méthyl]-3-aminopropanoïque :

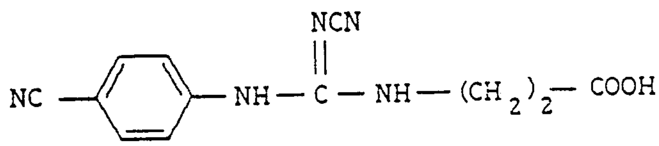

Un mélange de 32 g (0,2 mol) de 4-cyanophényl isothiocyanate et de 12,8 g (0,2 mol) de monosodium cyanamide dans 100 $cm^3$ d'éthanol absolu est maintenu durant 2 heures à l'ébullition. Après refroidissement, le précipité obtenu est filtré et lavé par 200 $cm^3$ d'éthanol absolu. Le solide est alors mis en suspension dans une solution de diméthyl sulfate (25 g, soit 0,2 mol) dans 500 $cm^3$ d'éthanol ; le mélange est chauffé durant deux heures à ébullition. Le précipité final est filtré, lavé par 2 × 100 $cm^3$ d'eau et par 2 × 100 $cm^3$ d'éthanol, puis est séché sous vide. On obtient 32,8 g (rendement 70 %) de N-(4-cyanophényl)-N'-cyano-S-méthylisothiourée :

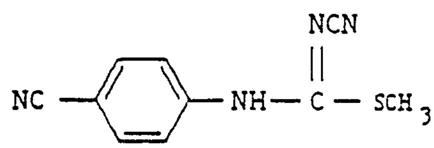

qui est un solide blanc dont le point de fusion est de 225-230 °C.

Un mélange de 12,3 g (0,138 mol) de β-alanine et de 5,52 g (0,138 mol) d'hydroxyde de sodium dans 100 $cm^3$ d'eau est ajouté à une solution de 20 g (0,092 mol) de N-(4-cyanophényl)-N'-cyano-S-méthylisothiourée dans 300 $cm^3$ d'éthanol à 95 %. Le mélange est chauffé au reflux durant 4 heures. Après refroidissement, la solution est filtrée puis concentrée à sec sous vide. Le résidu obtenu est dissous dans 200 $cm^3$ d'une solution de carbonate de sodium à 2 %. La solution résultante est lavée par du dichlorométhane (3 × 50 $cm^3$) puis acidifiée par une solution de HCl 3N jusqu'à l'obtention d'un pH voisin de 2. Le solide blanc formé est filtré, puis lavé par l'eau (2 × 10 $cm^3$) et séché sous vide. Après recristallisation dans l'eau, on obtient 15 g (rendement 63 %) d'acide N-[cyanoimino(4-cyanophénylamino)méthyl]-3-aminopropanoïque, qui est un solide dont le point de fusion est de 158-162 °C.

Les sels de sodium, de potassium et de calcium de cet acide ont été obtenus par dissolution de 1 g de l'acide dans 10 $cm^3$ d'eau contenant respectivement 0,15 g de NaOH, 0,21 g de KOH, 0,14 g de $Ca(OH)_2$. Après concentration à sec et recristallisation dans l'eau, les sels obtenus ont un point de fusion de 261 °C pour le sel de sodium, 260 °C pour le sel de potassium, de 236 °C pour le sel de calcium.

Ces composés, aussi bien l'acide que les sels, ont tous une intense saveur sucrée, tout à fait comparable à celle du saccharose, sans aucun arrière-goût. Leur pouvoir sucrant correspond approximativement, sur une base pondérale, à 900 (neuf cents) fois celui du saccharose comparativement à une solution de saccharose à 2 %, à 500 (cinq cents) fois par rapport à une solution de saccharose à 5 %, à 400 (quatre cents) fois par rapport à une solution de saccharose à 10 %.

## Exemple 2

Synthèse de l'acide N-[cyanoimino(4-cyanophénylamino) méthyl]-2-aminoéthanoïque :

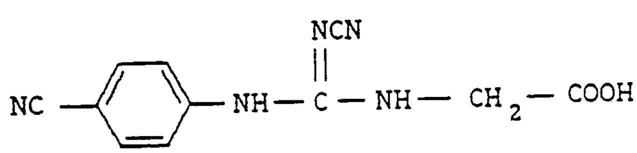

Ce composé est obtenu à partir de la glycine et de la N-(4-cyanophényl)-N'-cyano-S-méthylisothiourée, en suivant le protocole expérimental décrit dans l'exemple 1 (rendement 70 % ; point de fusion 103-105 °C, acétone-dichlorométhane). Son sel de sodium a un point de fusion de 178 °C ($H_2O$), son sel de calcium de 184 °C ($H_2O$).

Ces composés ont une intense saveur sucrée. Leur pouvoir sucrant correspond approximativement, sur une base pondérale, à 7 000 (sept mille) fois celui du saccharose par rapport à une solution de saccharose à 2 %, à 5 000 (cinq mille) fois par rapport à une solution de saccharose à 5 %, à 2 000 (deux mille) par rapport à une solution de saccharose à 10 %.

## Exemple 3

Synthèse de l'acide N-[cyanoimino(4-nitrophényl-amino) méthyl]-3-aminopropanoïque :

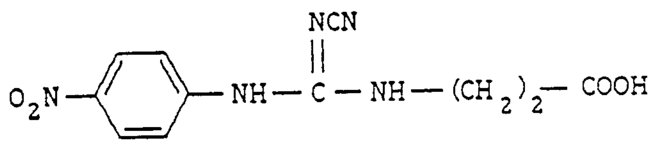

Ce composé est obtenu à partir de la β-alanine et de la N-(4-nitrophényl)-N'-cyano-S-méthylisothiourée, en suivant le protocole expérimental décrit dans l'exemple 1 (rendement 64 % ; point de fusion 165 °C à partir de l'eau).

Le pouvoir sucrant de ce composé correspond approximativement, sur une base pondérale, à 900 (neuf cents) fois celui du saccharose par rapport à une solution de saccharose à 2 %, à 500 (cinq cents) fois par rapport à une solution de saccharose à 5 %, à 400 (quatre cents) fois par rapport à une solution de saccharose à 10 %.

### Exemple 4

Synthèse de l'acide N-[2,2-dicyano(4-cyanophényl-amino) éthényl]-3-aminopropanoïque :

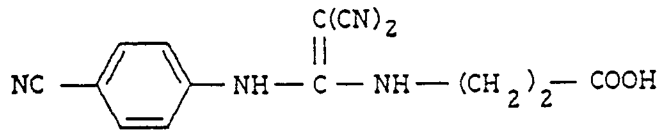

On ajoute 2,88 g d'hydrure de sodium (en dispersion à 50 % dans la paraffine liquide), en plusieurs fractions, à une solution, refroidie à 0 °C, de 3,96 g (0,06 mol) de malononitrile dissous dans 30 $cm^3$ de diméthylformamide. La solution est ensuite maintenue 10 minutes à 10 °C avant l'addition de 9,6 g (0,06 mol) de 4-cyanophényl isothiocyanate dissous dans 20 $cm^3$ de diméthylformamide. Le mélange réactionnel est maintenu durant 15 minutes à 20 °C puis concentré à sec sous vide. Le résidu obtenu est trituré à chaud dans du chloroforme (5 × 50 $cm^3$) et le solide final obtenu est séché sous vide. On met ensuite en contact 14 g du solide ainsi obtenu durant 2 heures à 20 °C avec 7,8 g de diméthyl sulfate dissous dans 200 $cm^3$ d'éthanol à 95 %. Après élimination de l'éthanol et lavage du solide restant par l'eau (4 × 50 $cm^3$), on obtient, après séchage, 9,4 g (rendement 70 %) de 1,1-dicyano-2-(4-cyanophénylamino)-2-(méthylthio)-éthène (point de fusion 160 °C) :

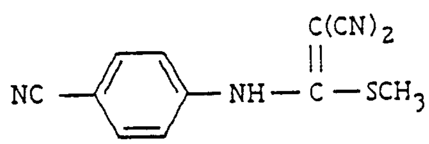

On mélange ensuite 12,5 g (0,14 mol) de β-alanine, 24 g (0,1 mol) du composé précédemment obtenu et 5,6 g (0,14 mol) d'hydroxyde de sodium dans une solution de 200 $cm^3$ d'éthanol chauffée à l'ébullition. Le mélange réactionnel est concentré à sec après 4 heures de contact et le résidu obtenu est dissous dans 200 $cm^3$ d'une solution aqueuse de carbonate de sodium à 2 %. La solution est lavée par du dichlorométhane (3 × 50 $cm^3$) puis acidifiée par une solution d'HCl 3N. Le précipité obtenu est filtré puis lavé par l'eau (2 × 10 $cm^3$). On obtient 15 g (rendement 55 %) d'acide N-[2,2-dicyano(4-cyanophénylamino) éthényl]-3-aminopropanoïque, qui est un solide blanc dont le point de fusion est de 201 °C (acétone-hexane).

Le pouvoir sucrant de ce composé correspond approximativement, sur une base pondérale à 500 (cinq cents) fois celui du saccharose par rapport à une solution de saccharose à 2 %, à 120 (cent vingt) fois par rapport à une solution de saccharose à 5 %, à 60 (soixante) fois par rapport à une solution de saccharose à 10 %.

### Exemple 5

Synthèse de l'acide N-[2,2-dicyano(4-cyanophényl-amino) éthényl]-2-aminoéthanoïque :

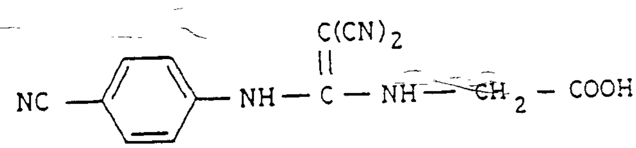

Ce composé est obtenu à partir de la glycine et du 1,1-dicyano-2-(4-cyanophénylamino)-2-(méthylthio)-éthène, en suivant le protocole expérimental décrit dans l'exemple 4 (rendement 50 % ; point de fusion 100 °C, acétone-dichlorométhane).

Le pouvoir sucrant de ce composé correspond approximativement, sur une base pondérale, à 4 000 (quatre mille) fois celui du saccharose par rapport à une solution de saccharose à 2 %, à 2 500 (deux mille cinq cents) fois par rapport à une solution de saccharose à 5 %, à 1 300 (mille trois cents) fois par rapport à une solution de saccharose à 10 %.

Exemple 6

Synthèse de l'acide N-[4-cyanophénylamino(imino) méthyl]-3-aminopropanoïque :

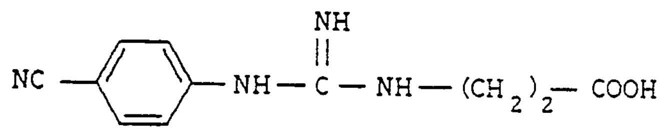

A une solution de 6,7 g (0,037 mol) de 4-cyano-phénylthiourée dans 150 cm³ de 2-butanone, on ajoute 13,1 g (0,092 mol) d'iodure de méthyle. Après 24 heures de contact à température ambiante, le solide formé est filtré, lavé par de la 2-butanone (2 × 20 cm³) puis par de l'éthyl éther (2 × 50 cm³), ce qui permet d'obtenir 9,2 g (rendement 76 %, point de fusion 212 °C) de N-(4-cyanophényl)-S-méthylisothiourée hydroiodure :

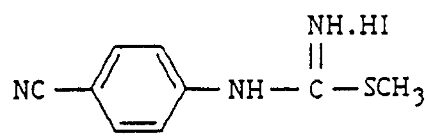

Un mélange de 4,18 g (47 mmol) de β-analine, de 1,88 g (47 mmol) d'hydroxyde de sodium dans 20 cm³ d'eau et de 15 g (47 mmol) de N-(4-cyanophényl)-S-méthylisothiourée hydroiodure dans 100 cm³ d'éthanol à 95 % est chauffé au reflux durant 5 heures. Le précipité formé est filtré, puis dissous dans 150 cm³ d'une solution aqueuse d'hydroxyde de sodium 1N. La solution obtenue est lavée par de l'acétate d'éthyle (3 × 50 cm³), puis amenée à pH 7 par une solution de HCl concentré. Le précipité obtenu est filtré, lavé par de l'éthanol absolu à chaud, puis recristallisé dans l'eau. Il est obtenu 7 g (rendement 65 %) d'acide N-[4-cyanophénylamino(imino)méthyl]-3-aminopropanoïque, dont le point de fusion est de 233-235 °C.

Le pouvoir sucrant de ce composé correspond approximativement, sur une base pondérale, à 300 (trois cents) fois celui du saccharose par rapport à une solution de saccharose à 2 %, à 180 (cent quatre-vingts) fois par rapport à une solution de saccharose à 5 %.

Exemple 7

Synthèse de l'acide N-[4-cyanophénylamino(imino)méthyl]-2-aminoéthanoïque :

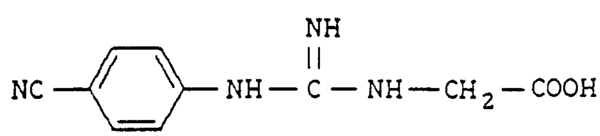

Ce composé est obtenu à partir de la glycine et du N-(4-cyanophényl)-S-méthylisothiourée hydroiodure, en suivant le protocole expérimental décrit dans l'exemple 6 (rendement 53 % ; point de fusion 275 °C, $H_2O$).

L'hydrochlorure de ce composé :

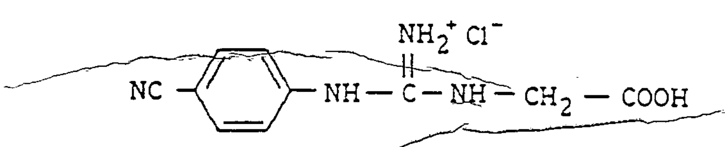

est obtenu par concentration à sec d'une solution de 1 g de l'acide N-[4-cyanophénylamino(imino)méthyl]-2-amino éthanoïque dans 15 cm³ d'une solution de HCl N. Son point de fusion est de 197 °C.

Le sel de sodium de l'acide M-[4-cyanophénylamino(imino)méthyl]-2-aminoéthanoïque est obtenu après dissolution de 1 g de l'acide N-[4-cyanophénylamino(imino)méthyl]-2-aminoéthanoïque dans 4,58 $cm^3$ d'une solution d'hydroxyde de sodium 1N suivie d'une concentration à sec. Le résidu obtenu est solubilisé dans l'acétone. La solution est filtrée, puis concentrée à sec, ce qui permet d'obtenir 1,1 g du sel de sodium correspondant (point de fusion 232 °C).

Le pouvoir sucrant de l'acide N-[4-cyanophénylamino(imino)méthyl]-2-aminoéthanoïque ou de ses sels (hydrochlorure ou sel de sodium) correspond approximativement, sur une base pondérale, à 2 700 (deux mille sept cents) fois celui du saccharose par rapport à une solution de saccharose à 2 %, à 1 600 (mille six cents) fois par rapport à une solution de saccharose à 5 %, à 600 (six cents) fois par rapport à une solution de saccharose à 10 %.

Exemple 8

Synthèse de l'acide N-[méthoxyimino(4-cyanophényl-amino)méthyl]-3-aminopropanoïque :

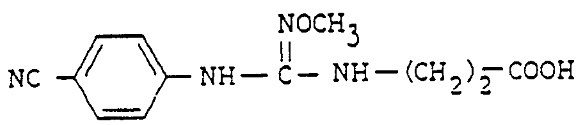

Ce composé est obtenu à partir de la β-alanine et de la N-(4-cyanophényl)-N'-méthoxy-S-méthylisothiourée, en suivant le protocole expérimental décrit dans l'exemple 1 (rendement 10 %, point de fusion 155 °C).

Le pouvoir sucrant de ce composé correspond approximativement, sur une base pondérale, à 300 (trois cents) fois celui du saccharose par rapport à une solution de saccharose à 2 %, à 100 (cent) par rapport à une solution de saccharose à 5 %, à 50 (cinquante) par rapport à une solution de saccharose à 10 %.

Exemple 9

Synthèse de l'acide N-[2-nitro(4-cyanophénylamino)éthényl]-2aminoéthanoïque :

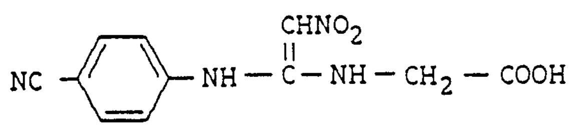

Un mélange de 8,3 g (0,05 mol) de 1-nitro-2,2-(méthylthio)-éthène et de 8,85 g (0,075 mol) de 4-aminobenzonitrile dans 40 $cm^3$ d'acide acétique glacial est chauffé au reflux durant 2 h. Après refroidissement le précipité formé est filtré, lavé par l'acide acétique glacial (5 $cm^3$), l'éthanol (2 × 10 $cm^3$), l'acétone (2 × 10 $cm^3$) et l'éthyl éther (2 × 10 $cm^3$). Le solide résultant est recristallisé dans le diméthylsulfoxyde. On obtient 3 g (rendement 30 %) de 1-nitro-2-(4-cyanophénylamino)-2-(méthylthio)-éthène dont le point de fusion est de 188-190 °C.

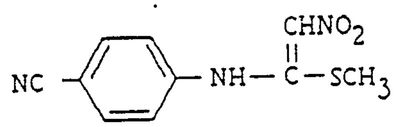

Un mélange de 0,15 g (2 mmol) de glycine, de 0,41 g (2 mmol) de 1-nitro-2-(4-cyanophénylamino)-2-(méthylthio)-éthène et de 0,27 $cm^3$ (2 mmol) de triéthylamine dans 20 $cm^3$ d'éthanol-eau (5-1), est chauffé au reflux durant 3 h. Après concentration sous vide, le résidu obtenu est dissous dans 20 $cm^3$ d'une solution 1N d'hydroxyde de sodium et la solution résultante est lavée par l'acétate d'éthyle (4 × 10 $cm^3$) puis acidifiée par une solution de chlorure d'hydrogène 6N jusqu'à obtention d'un pH voisin de 3.

Le solide blanc obtenu est filtré, lavé par l'eau (2 × 2 $cm^3$) et séché sous vide. On obtient 0,2 g (rendement 38 %) d'acide N-[2-nitro(4-cyanophenylamino)éthényl]-2-aminoéthanoïque dont le point de fusion est 230 °C.

Le pouvoir sucrant de ce composé correspond approximativement, sur une base pondérale, à 9 000 (neuf mille) fois celui du saccharose comparativement à une solution de saccharose à 2 %, à 6 000 (six mille) fois par rapport à une solution de saccharose à 5 %, à 2 700 (deux mille sept cents) fois par rapport à une solution de saccharose à 10 %.

Exemple 10

Synthèse de l'acide N-[phénylsulfonylimino(4-cyanophénylamino) méthyl]-2-aminoéthanoïque :

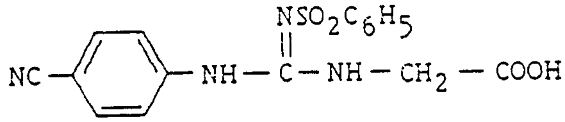

Un mélange de 5 g (0,031 mol) de 4-cyanophényl isothiocyanate, de 6,3 g (0,04 mol) de benzènesulfonamide dans 50 $cm^3$ d'acétone et de 1,6 g (0,04 mol) d'hydroxyde de sodium dans 3 $cm^3$ d'eau, est maintenu à température ambiante durant 2 heures. Le précipité formé est filtré, lavé par l'acétone et l'éthyl éther (2 × 20 $cm^3$). Le solide (9 g ; rendement 94 %) est alors placé dans 50 $cm^3$ d'éthanol à 95 % contenant 2,75 $cm^3$ d'iodure de méthyle (0,044 mol). Après 24 heures à température ambiante, la solution est évaporée à sec et le résidu est lavé par l'éthyl éther (3 × 20 $cm^3$) puis séché sous vide. On obtient 8,5 g (rendement 80 %) de N-(4-cyanophényl)-N'-phénylsulfonyl-S-méthylisothiourée dont le point de fusion est 150 °C :

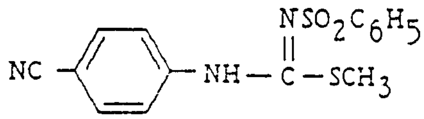

Un mélange de 0,57 g (7,6 mmol) de glycine, de 0,30 g (7,6 mmol) d'hydroxyde de sodium dans 3 $cm^3$ d'eau et de 2,5 g (7,6 mmol) de N-(4-cyanophényl-N'-phénylsulfonyl-S-méthylisothiourée dans 30 $cm^3$ d'éthanol à 95 %, est chauffé au reflux durant 7 h. Après refroidissement, le précipité obtenu est filtré puis dissous dans 20 $cm^3$ d'une solution 1N d'hydroxyde de sodium. La solution obtenue est lavée par le dichlorométhane (3 × 10 $cm^3$) et l'acétate d'éthyle (2 × 10 $cm^3$), puis acidifiée par addition d'une solution 6N d'acide chlorhydrique jusqu'à obtention d'un pH voisin de 3. Après refroidissement, le précipité obtenu est filtré, lavé par l'eau (2 × 5 $cm^3$) et séché sous vide pour donner 1,4 g (rendement 52 %) d'acide N-[phénylsulfonylimino(4-cyanophénylamino) méthyl]-2-aminoéthanoïque, dont le point de fusion est de 133 °C.

Le pouvoir sucrant de ce composé correspond approximativement, sur une base pondérale, à 45 000 (quarante cinq mille) fois celui du saccharose comparativement à une solution de saccharose à 2 %, à 25 000 (vingt-cinq mille) fois par rapport à une solution de saccharose à 5 %, et à 15 000 (quinze mille) par rapport à une solution de saccharose à 10 %.

Exemple 11

Synthèse de l'acide N-[phénylsulfonylimino(4-acétyl-phénylamino) méthyl]-2-aminoéthanoïque :

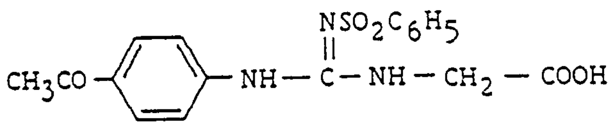

Ce composé est obtenu à partir de la glycine et de la N-(4-acétylphényl)-N'-phénylsulfonyl-S-méthylisothiourée, suivant le protocole expérimental décrit dans l'exemple 10 (rendement 38 % ; point de fusion 171 °C).

Le pouvoir sucrant de ce composé correspond approximativement, sur une base pondérale, à 850 (huit cent cinquante) fois celui du saccharose comparativement à une solution de saccharose à 2 %, à 750 (sept cent cinquante) fois par rapport à une solution de saccharose à 5 %, à 550 (cinq cent cinquante) fois comparativement à une solution de saccharose à 10 %.

Exemple 12

Synthèse de l'acide N-[phénylsulfonylimino(4-chlorophénylamino) méthyl]-2-aminoéthanoïque :

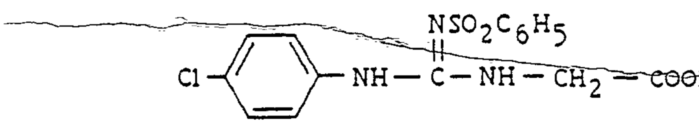

Ce composé est obtenu à partir de la glycine et de la N-(4-chlorophényl)-N'-phénylsulfonyl-S-méthylisothiourée, suivant le protocole expérimental décrit dans l'exemple 10 (rendement 30 % ; point de fusion 133 °C).

Le pouvoir sucrant de ce composé correspond approximativement, sur une base pondérale, à 2 000 (deux mille) fois celui du saccharose par rapport à une solution de saccharose à 2 %, à 1 400 (mille quatre cents) fois par rapport à une solution de saccharose à 5 %, à 750 (sept cent cinquante) fois par rapport à une solution de saccharose à 10 %.

Exemple 13

Synthèse de l'acide N-[méthylsulfonylimino(4-cyanophénylamino) méthyl]-2-aminoéthanoïque :

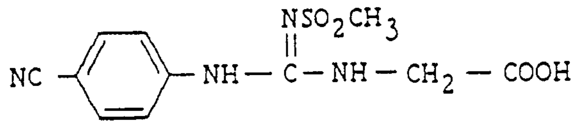

Ce composé est obtenu à partir de la glycine et de la N-(4-cyanophényl)-N'-méthylsulfonyl-S-méthylisothiourée, suivant le protocole expérimental décrit dans l'exemple 10 (rendement 55 % ; point de fusion 170 °C).

Le pouvoir sucrant de ce composé correspond approximativement, sur une base pondérale, à 4 000 (quatre mille) fois celui du saccharose par rapport à une solution de saccharose à 2 %, à 2 500 (deux mille cinq cents) fois par rapport à une solution de saccharose à 5 %, à 1 400 (mille quatre cents) fois par rapport à une solution de saccharose à 10 %.

Exemple 14

Synthèse de l'acide N-[phénylsulfonylimino(phénylamino) méthyl]-2-aminoéthanoïque :

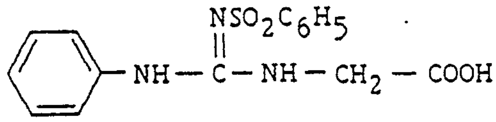

Ce composé est obtenu à partir de la glycine et de la N-phényl-N'-phénylsulfonyl-S-méthylisothiourée, suivant le protocole expérimental décrit dans l'exemple 10 (rendement 10 % ; point de fusion 148 °C).

Le pouvoir sucrant de ce composé correspond approximativement, sur une base pondérale, à 900 (neuf cents) fois celui du saccharose comparativement à une solution de saccharose à 2 %, à 800 (huit cents) fois par rapport à une solution de saccharose à 5 %, à 600 (six cents) fois comparativement à une solution de saccharose à 10 %.

Compte tenu des avantages déjà cités pour ces agents édulcorants, à savoir :

— pouvoir édulcorant élevé,
— pas d'arrière-goût amer ou réglissé,
— stabilité en solution,

et compte tenu que ces composés sont des édulcorants non nutritifs, non fermentescibles et non cariogènes, ces composés peuvent être utilisés avec succès, seuls ou en association avec d'autres édulcorants, en les ajoutant en quantité efficace pour édulcorer de nombreux produits, comme par exemple :

— comme édulcorants non nutritifs de table (en tablettes, paquets, etc.),
— dans les aliments diététiques et basse calorie, comme par exemple dans les boissons gazeuses (cola, limonades et boissons fruitées, bières, etc.) ou non gazeuses (jus de fruits ou de légumes, sirops, café, thé, cholocat, etc.) et les concentrés de boisson ou boissons instantanées en poudre, dans les cafés, thés, chocolats instantanés préédulcorés, dans les produits laitiers (laits et yogourts, laits en poudre préédulcorés, crèmes fouettées, etc.) ou analogues laitiers, dans les céréales et boissons préédulcorées pour petit-déjeuner, dans les desserts (desserts à la gélatine, puddings et autres gâteaux et pâtisseries) et dans les desserts congelés, ice creams et garnitures à la crème fouettée, dans les produits de boulangerie, dans les confitures, marmelades, gelées et miels diététiques, dans les assaisonnements, ketchups, pickles, sauces et autres arômes alimentaires, dans les confiseries (bonbons, bonbons à mâcher, confiseries au chocolat ou au cacao, pâtes de guimauve, etc.),
— dans les chewing gums,
— dans les dentifrices et les bâtons à lèvres,
— dans les bains de bouches et les gargarismes,
— dans diverses préparations pharmaceutiques, vétérinaires et cosmétiques (pour améliorer le goût de la préparation ou pour masquer le goût déplaisant de certains produits),
— dans divers articles d'hygiène,
— dans les tabacs,
— dans les aliments pour animaux, etc.

Les agents édulcorants de la présente invention peuvent être avantageusement utilisés sous forme de mélange avec un support compatible, comme par exemple, et de manière non limitative, l'amidon et les malto-dextrines, la cellulose, la méthylcellulose, la carboxyméthylcellulose, l'alginate de sodium, les pectines, les gommes, le lactose, le maltose, le glucose, la leucine, le glycérol, le mannitol, le sorbitol, les acides phosphoriques, citrique, tartrique, fumarique, benzoïque, sorbique, propionique, leurs sels de sodium, potassium et calcium, le bicarbonate de sodium.

Les agents édulcorants de la présente invention peuvent aussi être utilisés en association avec d'autres agents édulcorants comme par exemple le saccharose, le sirop de maïs, le fructose, l'aspartame, la glycyrrhizine, le xylitol, l'acésulfame-K, la thaumatine.

**Revendications** (pour les Etats contractants : BE, CH, DE, FR, GB, IT, LI, LU, NL, SE)

1. Agents édulcorants caractérisés en ce qu'ils répondent à la formule générale suivante :

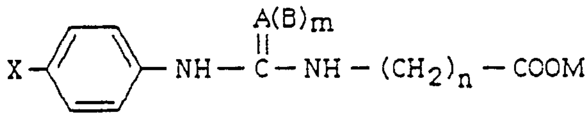

dans laquelle :

— A est un groupe imino (=N—), iminium ($=\overset{+}{N}<$), méthylène ($=C<$), le groupe iminium pouvant être salifié par un anion organique ou inorganique physiologiquement acceptable ;

— m et n sont des nombres égaux à 1 ou 2 ;

— B :

quand n = 1, représente H, CN, $OCH_3$, $NO_2$, $SO_2R$, SOR, $SO_2NHR$, $SO_2NR_2$, où R est un groupe alkyle, cycloalkyle ou aryle ayant jusqu'à 10 atomes de carbone, 1 ou 2 atomes de carbone pouvant être substitués par 1 ou 2 atomes de soufre ou d'oxygène ;

quand n = 2, représente H, CN, $OCH_3$ ;

— X :

représente CN, $NO_2$, quand B est H, CN, $OCH_3$ ;

représente CN, $NO_2$, $COCH_3$, CHO, Cl, $CF_3$, F, H quand B est $NO_2$, $SO_2R$ SOR, $SO_2NHR$, $SO_2NR_2$ ;

— M est un atome d'hydrogène ou un cation organique ou inorganique physiologiquement acceptable.

2. Agents édulcorants suivant la revendication 1 caractérisés en ce que :

— A est un groupe imino (=N—), iminium ($=\overset{+}{N}<$), méthylène ($=C<$), le groupe iminium pouvant être salifié par un ion chlorure ;

— B :

quand n = 1, représente H, CN, $OCH_3$, $NO_2$, $SO_2R$, où R est un groupe alkyle normal ou ramifié jusqu'à 5 atomes de carbone, isohexyle, phényle ($C_6H_5$), cyclohexyle ($C_6H_{11}$), benzyle ($C_6H_5CH_2$), tolyle ($CH_3C_6H_4$), cyclohexylméthyle ($C_6H_{11}CH_2$) ;

quand n = 2, représente H, CN, $OCH_3$ ;

— X est CN ;

— M est un atome d'hydrogène ou un cation pris dans le groupe $Na^+$, $K^+$, $NH_4^+$, 1/2 $Ca^{2+}$, 1/2 $Mg^{2+}$.

3. Agent édulcorant suivant la revendication 2 caractérisé en ce qu'il consiste en un composé de formule :

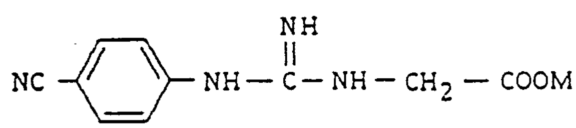

4. Agent édulcorant suivant la revendication 2 caractérisé en ce qu'il consiste en un composé de formule :

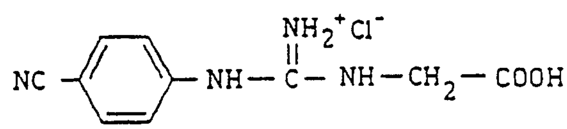

5. Agent édulcorant suivant la revendication 2 caractérisé en ce qu'il consiste en un composé de formule :

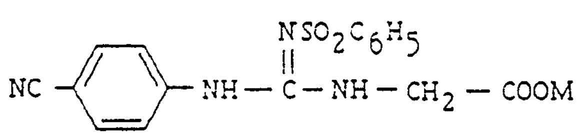

6. Agent édulcorant suivant la revendication 2 caractérisé en ce qu'il consiste en un composé de formule :

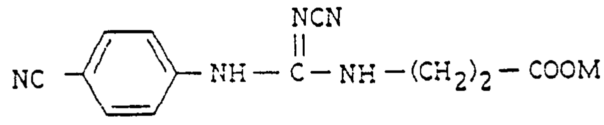

7. Procédé pour édulcorer les aliments, boissons, confiseries, chewing gums, articles de toilette, produits cosmétiques et d'hygiène, préparations pharmaceutiques et vétérinaires, caractérisé en ce qu'il consiste à y ajouter une quantité efficace d'un agent édulcorant selon l'une des revendications 1 à 6.

8. Préparations édulcorées suivant le procédé de la revendication 7.

9. Compositions édulcorantes caractérisées en ce qu'elles comprennent une quantité efficace d'un agent édulcorant selon l'une des revendications 1 à 6 et un support compatible, le support compatible étant choisi dans le groupe comprenant l'amidon et les malto-dextrines, la cellulose, la méthylcellulose et la carboxyméthylcellulose, l'alginate de sodium, les pectines, les gommes, le lactose, le maltose et le glucose, la leucine, le glycérol, le mannitol et le sorbitol, les acides phosphorique, citrique, tartrique, fumarique, benzoïque sorbique, propionique et leurs sels de sodium, potassium et calcium, le bicarbonate de sodium.

10. Compositions édulcorantes caractérisées en ce qu'elles comprennent un agent édulcorant selon l'une des revendications 1 à 6 et un autre agent édulcorant, l'autre agent édulcorant étant choisi dans le groupe comprenant le saccharose, le sirop de maïs, le fructose, l'aspartame, la glycyrrhizine, le xylitol, l'acésulfame-K, la thaumatine.

**Revendications** (pour l'Etat contractant AT)

1. Procédé pour la préparation d'agents édulcorants de formule générale suivante :

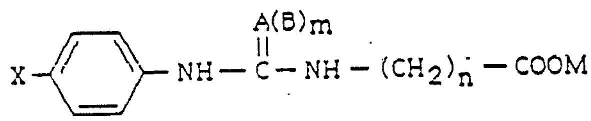

dans laquelle :

— A est un groupe imino (=N—), iminium ($=\overset{+}{N}<$), méthylène ($=C<$), le groupe iminium pouvant être salifié par un anion organique ou inorganique physiologiquement acceptable ;

— m et n sont des nombres égaux à 1 ou 2 ;

— B :

quand n = 1, représente H, CN, $OCH_3$, $NO_2$, $SO_2R$, SOR, $SO_2NHR$, $SO_2NR_2$, où R est un groupe alkyle, cycloalkyle ou aryle ayant jusqu'à 10 atomes de carbone, 1 ou 2 atomes de carbone pouvant être substitués par 1 ou 2 atomes de soufre ou d'oxygène,

quand n = 2, représente H, CN, $OCH_3$ ;

— X :

représente CN, $NO_2$, quand B est H, CN, $OCH_3$,

représente CN, $NO_2$, $COCH_3$, CHO, Cl, $CF_3$, F, H, quand B est $NO_2$, $SO_2R$, SOR, $SO_2NHR$, $SO_2NR_2$ ;

— M est un atome d'hydrogène ou un cation organique ou inorganique physiologiquement acceptable, caractérisé en ce qu'il consiste à condenser un premier composé de formule générale :

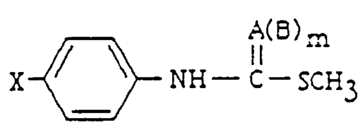

avec un composé de formule générale :

$$H_2N—(CH_2)_n—COOM$$

dans lesquelles X, A, B, m, n et M répondent aux définitions données ci-dessus.

2. Procédé selon la revendication 1 caractérisé en ce que dans les composés utilisés :

— A est un groupe imino (=N—), iminium ($=\overset{+}{N}<$), méthylène ($=C<$), le groupe iminium pouvant être salifié par un ion chlorure ;

— B :

quand n = 1, représente H, CN, $OCH_3$, $NO_2$, $SO_2R$, où R est un groupe alkyle normal ou ramifié jusqu'à 5 atomes de carbone, isohexyle, phényle ($C_6H_5$), cyclohexyle ($C_6H_{11}$), benzyle ($C_6H_5CH_2$), tolyle ($CH_3C_6H_4$), cyclohexylméthyle ($C_6H_{11}CH_2$),

quand n = 2, représente H, CN, $OCH_3$ ;

— X est CN ;

— M est un atome d'hydrogène ou un cation pris dans le groupe $Na^-$, $K^-$, $NH_4^-$, 1/2 $Ca^{2-}$, 1/2 $Mg^{2-}$.

3. Procédé selon la revendication 1, caractérisé en ce que la condensation est effectuée en présence d'une base, la base étant choisie dans le groupe comprenant l'hydroxyde de sodium, l'hydroxyde de potassium, la triéthylamine.

4. Procédé selon la revendication 1 et 3 caractérisé en ce que la condensation est effectuée à ébullition dans un mélange éthanol-eau.

5. Procédé de préparation, suivant la revendication 1, d'un agent édulcorant caractérisé en ce qu'il consiste en un composé de formule :

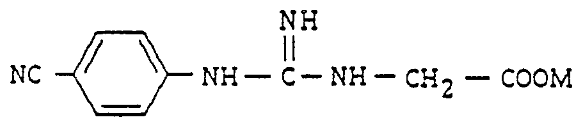

6. Procédé de préparation, suivant la revendication 1, d'un agent édulcorant caractérisé en ce qu'il consiste en un composé de formule :

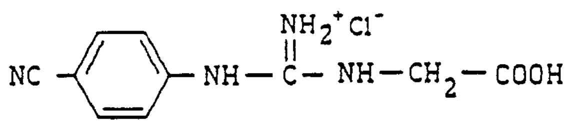

7. Procédé de préparation, suivant la revendication 1, d'un agent édulcorant caractérisé en ce qu'il consiste en un composé de formule :

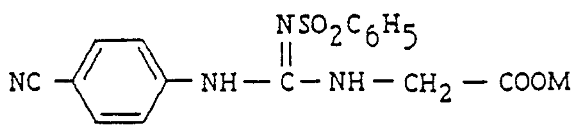

8. Procédé de préparation, suivant la revendication 1, d'un agent édulcorant caractérisé en ce qu'il consiste en un composé de formule :

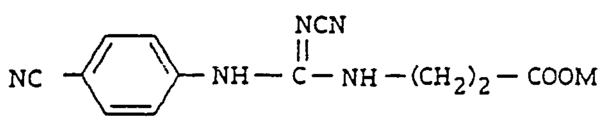

9. Procédé de préparation d'un produit consommable sucré, caractérisé en ce qu'il consiste à ajouter à ce produit une quantité efficace d'un agent édulcorant préparé selon l'une des revendications 1, 2, 5, 6, 7 et 8.

**Claims** (for the Contracting States : DE, FR, GB, IT, LI, LU, NL, SE)

1. Sweetening agents, characterized in that they respond to the following general formula :

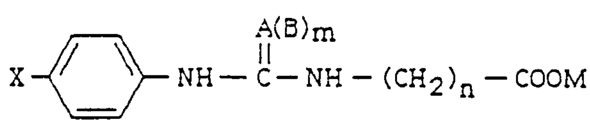

wherein :

— A is an imino (=N—), iminium ($=\overset{+}{N}<$), methylene (=C < ) group, the iminium group being able to be salified by a physiologically acceptable organic or inorganic anion ;

— m and n are numbers equal to 1 or 2 ;

— B :

when n = 1, represents H, CN, $OCH_3$, $NO_2$, $SO_2R$, SOR, $SO_2NHR$, $SO_2NR_2$, wherein R is an alkyl, cycloalkyl or aryl group having up to 10 carbon atoms, 1 or 2 carbon atoms being able to be substituted by 1 or 2 sulfur or oxygen atoms ;

when n = 2, represents H, CN, $OCH_3$ ;

— X :

represents CN, $NO_2$, when B is H, CN, $OCH_3$ ;

represents CN, $NO_2$, $COCH_3$, CHO, Cl, $CF_3$, F, H, when B is $NO_2$, $SO_2R$, SOR, $SO_2NHR$, $SO_2NR_2$ ;

— M is a hydrogen atom or a physiologically acceptable organic or inorganic cation.

2. Sweetening agents according to claim 1 characterized in that :

— A is an imino (=N—), iminium ($=\overset{+}{N} <$ ), methylene (=C < ) group, the iminium group being able to be salified by a chloride ion ;

— B :

when n = 1, represents H, CN, $OCH_3$, $NO_2$, $SO_2R$, wherein R is a normal or branched alkyl group having up to 5 carbon atoms, isohexyl, phenyl ($C_6H_5$), cyclohexyl ($C_6H_{11}$), benzyl ($C_6H_5CH_2$), tolyl ($CH_3C_6H_4$), cyclohexylmethyl ($C_6H_{11}CH_2$) ;

when n = 2, represents H, CN, $OCH_3$ ;

— X is CN ;

— M is a hydrogen atom or a cation selected from the group $Na^+$, $K^+$, $NH_4^+$, 1/2 $Ca^{2+}$, 1/2 $Mg^{2+}$.

3. Sweetening agent according to claim 2 characterized in that it consists in a compound of formula :

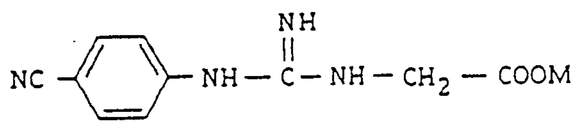

4. Sweetening agent according to claim 2 characterized in that it consists in a compound of formula :

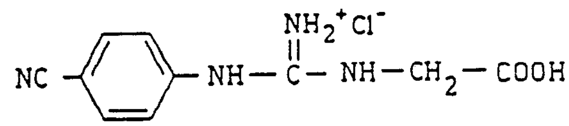

5. Sweetening agent according to claim 2 characterized in that it consists in a compound of formula :

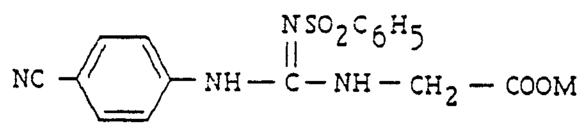

6. Sweetening agent according to claim 2 characterized in that it consists in a compound of formula :

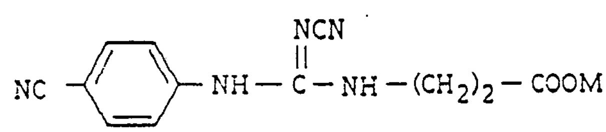

7. Process for sweeten foods, beverages, confectionery, chewing gums, toilet, cosmetic and hygiene products, pharmaceuticals and veterinary preparations, characterized in that it consists in adding an effective amount of a sweetening agent according to one of the claims 1 to 6.

8. Preparations sweetened according to the process of claims 7.

9. Sweetening compositions characterized in that they comprise an effective amount of a sweetening agent according to one of the claims 1 to 6 and a carrier therefor, the carrier being chosen in the group comprising starch and malto-dextrins, cellulose, methylcellulose and carboxymethylcellulose, sodium alginate, pectins, gums, lactose, maltose and glucose, leucine, glycerol, mannitol and sorbitol, phosphoric, citric, tartaric, fumaric, benzoic, sorbic, propionic acids and their sodium, potassium and calcium salts, sodium bicarbonate.

10. Sweetening compositions characterized in that they comprise a sweetening agent according to one of the claims 1 to 6 and another sweetening agent, the other sweetening agent being chosen in the group comprising sucrose, corn syrups, fructose, aspartame, glycyrrhizin, xylitol, acesulfame-K, thaumatin.

**Claims** (for the Contracting State AT)

1. Process for manufacturing sweetening agents responding to the following general formula :

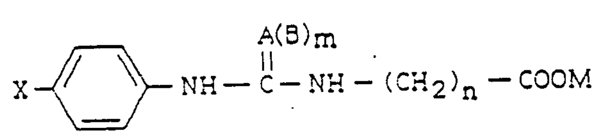

wherein :

— A is an imino (=N—), iminium ($=\overset{+}{N}<$ ), methylene (=C < ) group, the iminium group being able to be salified by a physiologically acceptable organic or inorganic anion ;

— m and n are numbers equal to 1 or 2 ;

— B :

when n = 1, represents H, CN, $OCH_3$, $NO_2$, $SO_2R$, SOR, $SO_2NHR$, $SO_2NR_2$, wherein R is an alkyl, cycloalkyl or aryl group having up to 10 carbon atoms, 1 or 2 carbon atoms being able to be substituted by 1 or 2 sulfur or oxygen atoms ;

when n = 2, represents H, CN, $OCH_3$ ;

— X :

represents CN, $NO_2$, when B is H, CN, $OCH_3$ ;

represents CH, $NO_2$, $COCH_3$, CHO, Cl, $CF_3$, F, H, when B is $NO_2$, $SO_2R$, SOR, $SO_2NHR$, $SO_2NR_2$ ;

— M is a hydrogen atom or a physiologically acceptable organic or inorganic cation characterized in the fact that it consists in condensating a first compound of general formula :

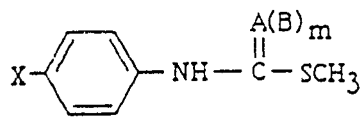

with a compound of general formula :

$$H_2N—(CH_2)_n—COOM$$

wherein X, A, B, m, n and M correspond to the above definitions.

2. Process according to claim 1, wherein :

— A is an imino (=N—), iminium ($=\overset{+}{N}<$ ), methylene (=C < ) group, the iminium group being able to be salified by a chloride ion ;

— B :

when n = 1, represents H, CN, $OCH_3$, $NO_2$, $SO_2R$, wherein R is a normal or branched alkyl group having up to 5 carbon atoms, isohexyl, phenyl ($C_6H_5$), cyclohexyl ($C_6H_{11}$), benzyl ($C_6H_5CH_2$), tolyl ($CH_3C_6H_4$), cyclohexylmethyl ($C_6H_{11}CH_2$) ;

when n = 2, represents H, CN, $OCH_3$ ;

— X is CN ;

— M is a hydrogen atom or a cation selected from the group $Na^+$, $K^+$, $NH_4^+$, 1/2 $Ca^{2+}$, 1/2 $Mg^{2+}$.

3. Process according to claim 1, wherein the condensation is carried out in the presence of a base selected in the group comprising sodium hydroxide, potassium hydroxide, and triethylamine.

4. Process according to claims 1 and 3, wherein the condensation is carried out at boiling point in an ethanol-water mixture.

5. Process according to claim 1 for manufacturing a sweetening agent, wherein said sweetening agent consists in a compound of formula :

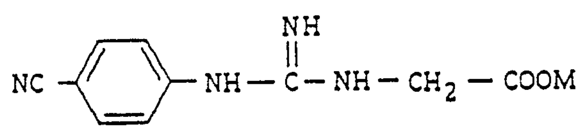

6. Process according to claim 1 for manufacturing a sweetening agent, wherein said sweetening agent consists in a compound of formula :

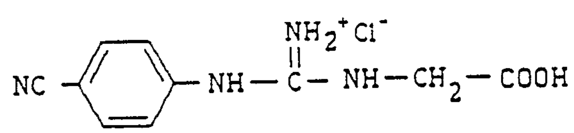

7. Process according to claim 1 for manufacturing a sweetening agent, wherein said sweetening agent consists in a compound of formula :

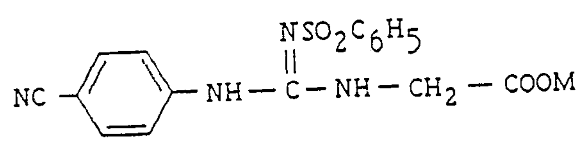

8. Process according to claim 1, for manufacturing a sweetening agent, wherein said sweetening agent consists in a compound of formula :

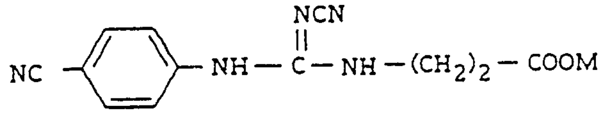

9. Process for manufacturing a sweetener consumable product, characterized in the fact that it consists in adding an effective amount of a sweetening agent manufactured according to one of the claims 1, 2, 5, 6, 7 and 8.

**Patentansprüche** (für die Vertragsstaaten : DE, FR, GB, IT, LI, LU, NL, SE)

1. Süßstoffe, gekennzeichnet durch die allgemeine Formel

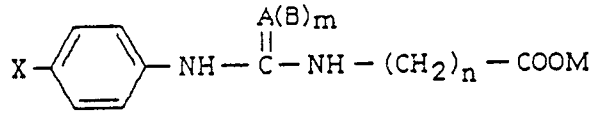

bei der :

— A eine Imino- (=N—), eine Iminium- ($=\overset{+}{N}$ ) oder eine Methylengruppe (=C ) ist, wobei die Iminiumgruppe mit einem physiologisch verträglichen, organischen oder anorganischen Anion ein Salz bildet ;

— m und n 1 oder 2 sind ;

— B :

falls n = 1 ist, H, CN, $OCH_3$, $NO_2$, $SO_2R$, SOR, $SO_2NHR$ oder $SO_2NR_2$ ist, worin R eine Alkyl-, eine Cycloalkyl- oder eine Arylgruppe mit bis zu 10 Kohlenstoffatomen ist, wobei 1 oder 2 Kohlenstoffatome durch 1 oder 2 Schwefel- oder Sauerstoffatome ersetzt sein können ;

falls n = 2 ist, H, CN oder $OCH_3$ ist ;

— X :

CN oder $NO_2$ ist, falls B H, CN oder $OCH_3$ ist ;

CN, $NO_2$, $COCH_3$, CHO, Cl, $CF_3$, F oder H ist, falls B $NO_2$, $SO_2R$, SOR, $SO_2NHR$ oder $SO_2NR_2$ ist ;

— M ein Wasserstoffatom oder ein physiologisch verträgliches organisches oder anorganisches Kation ist.

2. Süßstoffe nach Anspruch 1, dadurch gekennzeichnet, daß

— A eine Imino- (=N—), eine Iminium- ($=\overset{+}{N} <$ ) oder eine Methylengruppe ($=C <$ ) ist, wobei die Iminiumgruppe mit einem Chloridion ein Salz bildet ;

— B :

falls n = 1 ist, H, CN, $OCH_3$, $NO_2$ oder $SO_2R$ ist, worin R eine gerade oder verzweigte Alkylgruppe mit bis zu 5 Kohlenstoffatomen, eine Isohexyl-, Phenyl-($C_6H_5$), Cyclohexyl-($C_6H_{11}$), Benzyl-($C_6H_5CH_2$), Tolyl-($CH_3C_6H_4$) oder eine Cyclohexylmethylgruppe ($C_6H_{11}CH_2$) ist ;

falls n = 2 ist, H, CN oder $OCH_3$ ist ;

— X CN ist ; und

— M ein Wasserstoffatom oder ein Kation, ausgewählt aus der Gruppe bestehend aus $Na^-$, $K^-$, $NH_4^-$, 1/2 $Ca^{2-}$ oder 1/2 $Mg^{2-}$ ist.

3. Süßstoff nach Anspruch 2, dadurch gekennzeichnet, daß er aus einer Verbindung der Formel

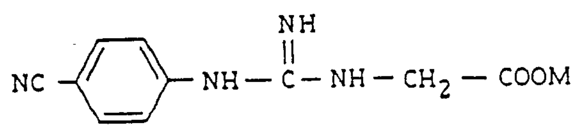

besteht.

4. Süßstoff nach Anspruch 2, dadurch gekennzeichnet, daß er aus einer Verbindung der Formel

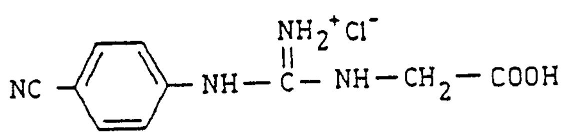

besteht.

5. Süßstoff nach Anspruch 2, dadurch gekennzeichnet, daß er aus einer Verbindung der Formel

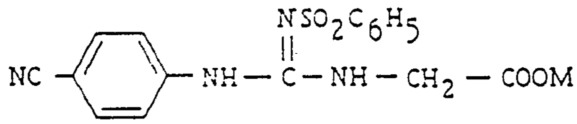

besteht.

6. Süßstoff nach Anspruch 2, dadurch gekennzeichnet, daß er aus einer Verbindung der Formel

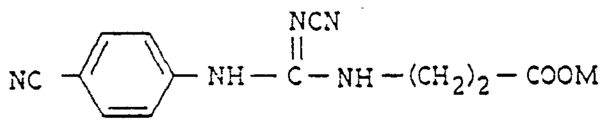

besteht.

7. Verfahren zum Süßen von Nahrungsmitteln, Getränken, Süßwaren, Kaugummis, Toilettenartikeln, kosmetischen und hygienischen Produkten, pharmazeutischen und veterinärischen Präparaten, dadurch gekennzeichnet, daß eine wirksame Menge eines Süßstoffes nach einem der Ansprüche 1 bis 6 zugefügt wird.

8. Gesüßte Erzeugnisse des Verfahrens nach Anspruch 7.

9. Süße Zusammensetzungen, dadurch gekennzeichnet, daß sie eine wirksame Menge eines Süßstoffes nach einem der Ansprüche 1 bis 6 und ein kompatibles Trägermaterial enthalten, wobei das kompatible Trägermaterial, ausgewählt aus der Gruppe bestehend aus Stärke und Malto-Dextrinen, Zellulose, Methylzellulose und Carboxymethylzellulose, Natriumalginat, Pektinen, Gummi, Lactose, Maltose, Glucose, Leuzin, Glycerol, Mannit, Sorbit, Phosphor-, Zitronen-, Wein-, Fumar-, Benzoe-, Sorbin-, Propionsäure und deren Natrium-, Kalium-, und Calziumsalze, und Natriumbicarbonat ist.

10. Süße Zusammensetzungen, dadurch gekennzeichnet, daß sie einen Süßstoff nach einem der Ansprüche 1 bis 6 und einen weiteren Süßstoff enthalten, wobei der weitere Süßstoff, ausgewählt aus der Gruppe bestehend aus Rohrzucker, Maissirup, Fruchtzucker, Aspartam, Glyzyrrhizin, Xylit, Acesulfam-K und Thaumatin ist.

**Patentansprüche** (für den Vertragsstaat AT)

1. Verfahren zum Herstellen von Süßstoffen mit der allgemeinen Formel

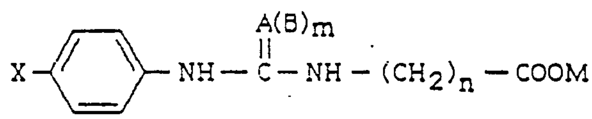

bei der :

— A eine Imino- (=N—), eine Iminium- ($=\overset{+}{N}<$ ) oder eine Methylengruppe (=C < ) ist, wobei die Iminiumgruppe mit einem physiologisch verträglichen, organischen oder anorganischen Anion ein Salz bildet ;

— m und n 1 oder 2 sind ;

— B :

falls n = 1 ist, H, CN, $OCH_3$, $NO_2$, $SO_2R$, SOR, $SO_2NHR$ oder $SO_2NR_2$ ist, worin R eine Alkyl-, eine Cycloalkyl- oder eine Arylgruppe mit bis zu 10 Kohlenstoffatomen ist, wobei 1 oder 2 Kohlenstoffatome durch 1 oder 2 Schwefel- oder Sauerstoffatome ersetzt sein können

falls n = 2 ist, H, CN oder $OCH_3$ ist ;

— X :

CN oder $NO_2$ ist, falls B H, CN oder $OCH_3$ ist ;

CN, $NO_2$, $COCH_3$, CHO, Cl, $CF_3$, F oder H ist, falls B $NO_2$, $SO_2R$, SOR, $SO_2NHR$ oder $SO_2NR_2$ ist ;

— M ein Wasserstoffatom oder ein physiologisch verträgliches organisches oder anorganisches Kation ist ; dadurch gekennzeichnet, daß eine erste Verbindung der allgemeinen Formel

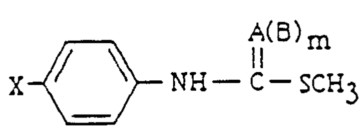

mit einer Verbindung der allgemeinen Formel

$$H_2N—(CH_2)_n—COOM$$

kondensiert wird, in denen X, A, B, m, n und M die oben gegebenen Bedeutungen haben.

2. Verfahren nach Anspruch 1, dadurch gekennzeichnet, daß Verbindungen verwendet werden, bei denen :

— A eine Imino- (=N—), eine Iminium- (=$\overset{+}{N}$ < ) oder eine Methylengruppe (=C < ) ist, wobei die Iminiumgruppe mit einem Chloridion ein Salz bilden kann ;

— B :

falls n = 1 ist, H, CN, $OCH_3$, $NO_2$ oder $SO_2R$ ist, worin R eine gerade over verzweigte Alkylgruppe mit bis zu 5 Kohlenstoffatomen, eine Isohexyl-, Phenyl-($C_6H_5$), Cyclohexyl-($C_6H_{11}$), Benzyl-($C_6H_5CH_2$), Tolyl-($CH_3C_6H_4$) oder eine Cyclohexylmethylgruppe ($C_6H_{11}CH_2$) ist ;

falls n = 2 ist, H, CN oder $OCH_3$ ist

— X CN ist ; und

— M ein Wasserstoffatom oder ein Kation, ausgewählt aus der Gruppe bestehend aus $Na^-$, $K^-$, $NH_4^-$, 1/2 $Ca^{2-}$ oder 1/2 $Mg^{2-}$ ist.

3. Verfahren nach Anspruch 1, dadurch gekennzeichnet, daß die Kondensation in Gegenwart einer Base durchgeführt wird, wobei die Base, ausgewählt aus der Gruppe bestehend aus Natriumhydroxid, Kaliumhydroxid und Triethylamin ist.

4. Verfahren nach den Ansprüchen 1 und 3, dadurch gekennzeichnet, daß die Kondensation in einem siedenden Ethanol/Wasser-Gemisch durchgeführt wird.

5. Verfahren zum Herstellen eines Süßstoffes nach Anspruch 1, dadurch gekennzeichnet, daß er aus einer Verbindung der Formel

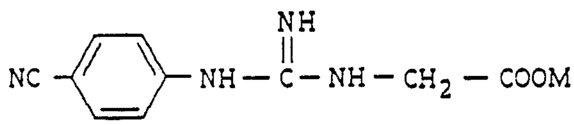

besteht.

6. Verfahren zum Herstellen eines Süßstoffes nach Anspruch 1, dadurch gekennzeichnet, daß er aus einer Verbindung der Formel

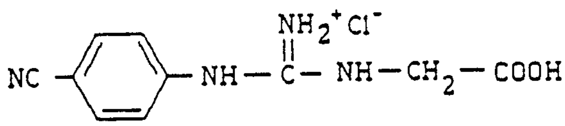

besteht.

7. Verfahren zum Herstellen eines Süßstoffes nach Anspruch 1, dadurch gekennzeichnet, daß er aus einer Verbindung der Formel

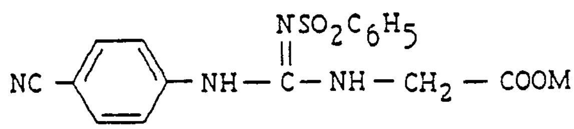

besteht.

8. Verfahren zum Herstellen eines Süßstoffes nach Anspruch 1, dadurch gekennzeichnet, daß er aus einer Verbindung der Formel

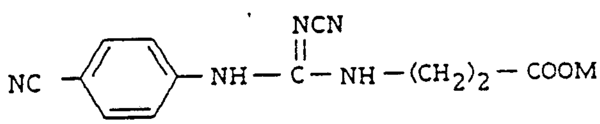

besteht.

9. Verfahren zum Herstellen eines genießbaren gesüßten Produktes, dadurch gekennzeichnet, daß diesem Produkt eine wirksame Menge eines Süßstoffes, der nach einem der Ansprüche 1, 2, 5, 6, 7 und 8 hergestellt wurde, zugefügt wird.